# EUROPÄISCHE PATENTANMELDUNG

(11) **EP 0 679 395 A1**
(43) Veröffentlichungstag der Anmeldung: **02.11.1995**
(21) Anmeldenummer: 95105828.8
(22) Anmeldetag: 19.04.1995
(51) Int. Cl.: A61K 31/19, A61K 9/48

(54) **Pharmazeutische Zubereitungen aud der Basis einer Ketoprofenlösung in weichen Gelatinekapseln und Verfahren zu deren Herstellung**

(30) Priorität: 26.04.1994 IT MI940801
(71) Anmelder: Bayer S.p.A., 20156 Milano (IT)
(72) Erfinder: Dondi, Gilberto, Dr., Cusano Milanino, Milano (IT); Scurati, Paolo, Dr., Paderno Dugnano, Milano (IT)
(74) Vertreter: Braun, Rolf, Dr.

(57) **Zusammenfassung**

Die vorliegende Erfindung betrifft eine stabile pharmazeutische Zubereitung, die Ketoprofen enthält, und insbesondere eine Zubereitung, die Ketoprofen in einem Träger enthält, wie z. B. Polyethylenglykol.

## Beschreibung

Die vorliegende Erfindung betrifft eine stabile pharmazeutische Zubereitung, die Ketoprofen enthält, und insbesondere eine Zubereitung, die Ketoprofen in einem Träger enthält, wie z.B. Polyethylenglykol.

Diese pharmazeutische Zubereitung bietet die Möglichkeit, über ein Arzneimittel mit einer sehr schnellen therapeutischen Wirkung zu verfügen, die besonders nützlich ist zur Verabreichung eines Wirkstoffes mit analgetischer Wirkung.

Ferner betrifft die vorliegende Erfindung auch ein Verfahren zur Herstellung derartiger pharmazeutischer Zubereitungen.

Es ist bekannt, daß Ketoprofen, d.h. 2-(3-Benzoylphenyl)propionsäure in der Medizin weitgehend als antiphlogistisches und analgetisches Mittel verwendet wird. Es ist verfügbar in verschiedenen Arzneiformen zur oralen, topischen, parenteralen Verwendung usw. Es bestand jedoch ein Bedürfnis, die Resorptionsgeschwindigkeit der Formen für orale Verabreichung beträchtlich zu erheben, um die biologische Verfügbarkeit des Arzneimittels aufs höchste zu beschleunigen, um eine sehr schnelle pharmakologische Wirkung zu erzielen.

Es ist deshalb Aufgabe vorliegender Erfindung, eine Arzneiform zu schaffen, die in der Lage ist, seitens des Organismus eine rasche Resorption des oral verabreichten Ketoprofens zu gewährleisten.

Gemäß vorliegender Erfindung, wird diese Aufgabe durch die im Hauptanspruch gekennzeichnete pharmazeutische Zubereitung gelöst.

Dem Fachmann sind zahlreiche Methoden bekannt, um einen Wirkstoff in weiche Gelatinkapseln einzubringen, beispielsweise das Einbringen des Wirkstoffs einer Dispersion in einem Träger auf Fettbasis, gegebenenfalls unter Zugabe von Tensiden.

Es ist jedoch bekannt, daß es oft vorzuziehen ist, Pharmaka als Lösung in Wasser oder in anderem hydrophilen Lösungsmittel zu verabreichen, da man somit die Auflösungsphase des Pharmakons im Verdauungsapparat vermeiden kann. Das ist besonders wünschenswert im Falle der Analgetika, da eine rasche pharmakologische Wirkung einen Wahlmaßstab darstellt. Ein weiterer Nachteil der Verwendung von Trägersubstanzen auf Fettbasis besteht darin, daß sie undurchsichtige Kapseln bilden von einem nicht angenehmen Aspekt, weshalb es unerläßlich ist, Pigmente und/oder Farbstoffe einzusetzen, um dem Mittel ästhetische Merkmale zu verleihen, die dazu verhelfen, die Akzeptanz seitens der Patienten zu verbessern. Es wurde nun gefunden, daß die Polyethylenglykole (PEG) mit einem Molekulargewicht zwischen 200 und 600 besonders geeignete Substanzen sind zur Zubereitung von Ketoprofenlösungen zum Einbringen in weiche Gelatinkapseln.

Gemäß vorliegender Erfindung löst man Ketoprofen in einem Polyethylenglykol mit einem Molekulargewicht zwischen 200 und 600 in einem Ketoprofen: PEG-Verhältnis zwischen 1:8 und 1:2.

Ein weiterer kritischer Maßstab innerhalb vorliegender Erfindung ist der pH-Wert der Ketoprofenlösung im Polyethylenglykol, um eine angemessene Stabilität der pharmazeutischen Zubereitung zu gewährleisten und deren Verträglichkeit zu verbesseren. Die besten Ergebnisse wurden erreicht für pH-Werte zwischen 5 und 7 . Gemäß vorliegender Erfindung, können diese pH-Werte erreicht werden mittels Zusatz von zweckmäßigen Neutralisationsmitteln, wie z.B. Colamin (Ethanolamin), Lysin, Triethanolamin, Methylglucamin und im allgemeinen jedes passende Amin vom Typ, wie er für die Zubereitung pharmazeutischer Zusammensetzungen verwendet wird.

Gemäß der Erfindung, wird die Herstellung von pharmazeutischen Zubereitungen auf der Basis von Ketoprofen in weichen Gelatinkapseln in zwei Stufen durchgeführt. Zuerst löst man, gemäß dem Fachmann bekannten Methoden, eine angemessene Gelatinmenge, Glyzerin und gereinigtes Wasser, indem man diese Zutaten bei einer Temperatur von zirka 80°C drei stundenlang unter ständigem Schütteln erhitzt.

Nach Entlüftung unter vermindertem Druck wird die so zubereitete Lösung bei zirka 50°C aufbewahrt zur Zubereitung von weichen Kapseln.

Danach löst man eine therapeutisch wirkende Menge Ketoprofen in einer angemessenen Menge PEG, wo nötig, stellt man den pH-Wert bis auf einen Wert zwischen 5 und 7 ein mit einer pharmazeutisch verträglichen Basis und schüttelt sie bis zum Erreichen einer durchsichtigen Lösung. Falls es als nützlich erachtet werden sollte, kann man dieser Lösung kleine Mengen sterilen Wassers, Propylenglykol, Glyzerin, Konservierungsmittel oder andere Zusatzstoffe hinzufügen, wie sie allgemein bei der Zubereitung von pharmazeutischen Zusammensetzungen in Form weicher Kapseln verwendet werden.

Schließlich wird die erfindungsgemäße Lösung eingekapselt, wobei man Methoden und Apparaturen verwendet, wie sie allgemein für die Zubereitung dieser speziellen pharmazeutischen Form gebraucht werden.

Die Erfindung wird näher erläutert mittels folgender Beispiele. Sobald die Grundprinzipien zur Verwirklichung der pharmazeutischen Zubereitungen vorliegender Erfindung erlernt sind, in Form von weichen Gelatinkapseln, wird der Fachmann pharmazeutischer Zubereitungen keinerlei Schwierigkeit haben, die Verfahrensmaßstäbe an die besonderen Notwendigkeiten anzupassen, ohne sich deshalb von dem Erfindungsgedanken zu entfernen.

### Beispiel 1

### Zubereitung von weichen Ketoprofenkapseln von je 25 mg

5000 weiche Gelatinkapseln, von denen jede 25 mg Ketoprofen enthält, wurden mit folgenden Zutaten zubereitet:

| | |
|---|---|
| Gelatine pro Kapsel | g 460,0 |
| Glyzerin | g 140,0 |
| Gereinigtes Wasser | g 330,0 |
| Ketoprofen | g 125,0 |
| PEG 600 | g 625,0 |
| Ethanolamin | g 28,1 |

Die Zubereitung der Gelatinhülle, die zur Vorbereitung der weichen Kapseln dient, ist für sich kein Teil vorliegender Erfindung, da dies bereits bekannt ist. Im allgemeinen wird die Zubereitung dieser Hülle von Firmen vorgenommen, die in der Herstellung weicher Gelatinkapseln spezialisiert sind.

Nachfolgend wird in großen Zügen die Zubereitung erläutert, mit dem einzigen Zweck, eine vollständiger Beschreibung der Erfindung zu liefern.

Die Gelatine wird in der vorerwähnten Menge mit Glyzerol und gereinigtem Wasser in den angegebenen Mengen gemischt, wobei die Mischung drei stundenlang bei 80°C unter ständigem Schütteln erhitzt wird. Nach Entlüftung unter vermindertem Druck wird die so erhaltene Lösung bei einer Temperatur von 50°C erhalten bis zur Zubereitung der Schicht für die Hülle. Wie es dem Fachmann bekannt ist, kann man dieser Lösung, wo als nötig erachtet, weitere Stoffe hinzufügen, wie z.B. Propylenglykol, Sorbit, Konservierungsmittel, Farbstoffe. Danach wird das Ketoprofen in PEG 600 gelöst unter Zusatz von Ethanolamin in den vorgenannten Mengen, und wird solange geschüttelt, bis man eine durchsichtige Lösung erhält; auf diese Weise haben die Bestandteile der pharmazeutischen Zubereitung der Erfindung eine der Verkapselung angemessene Form. Die einzelnen Kapseln, von denen jede 25 mg Ketoprofen enthält, werden dann so zubereitet, indem sie, in an sich bekannter Weise, 156 mg der oben beschriebenen Lösung verkapseln.

### Beispiel 2

### Zubereitung von weichen Kapseln mit je 50 mg Ketoprofen

2500 weiche Kapseln, die je 50 mg Ketoprofen enthalten, sind gemäß der Beschreibung in Beispiel 1 zubereitet worden, jedoch unter Verwendung von 312 mg Ketoprofenlösung anstatt 156 mg .

### Beispiel 3

Gemäß dem in Beispiel 1 beschriebenen Verfahren, löst man 125 g Ketoprofen in 625 g PEG 400 . Der pH-Wert der Lösung wird eingestellt mit 135 g 50%igem DL-Lysin, und es werden 5000 weiche Kapseln von je 25 mg Ketoprofen gemäß Beispiel 1 zubereitet.

### Beispiel 4

Gemäß dem in Beispiel 1 beschriebenen Verfahren löst man 125 g Ketoprofen in einer Mischung von 400 g PEG 600 und 125 g PEG 400 . Man stellt den pH-Wert der Lösung mit 27,9 g Ethanolamin richtig, und man bereitet weiche Kapseln zu, indem man in jeder 156 mg der so zubereiteten Lösung verkapselt, in welchem Falle die Dosierung pro Kapsel 25 mg therapeutisch wirkender Substanz betragen wird, wohingegen für die Zubereitung von Kapseln mit je 50 mg Ketoprofen 312 mg der gleichen Lösung verkapselt werden müssen.

### Beispiel 5

Gemäß dem in Beispiel 1 beschriebenen Verfahren, löst man 125 g Ketoprofen in einer Mischung von 600 g PEG 400 und 25 g Glyzerol. Man fügt 28 g Ethanolamin hinzu, und man verkapselt gemäß den in Beispiel 1 beschriebenen Bedingungen entsprechend der Dosierung 156 g oder 312 g der so zubereiteten Lösung.

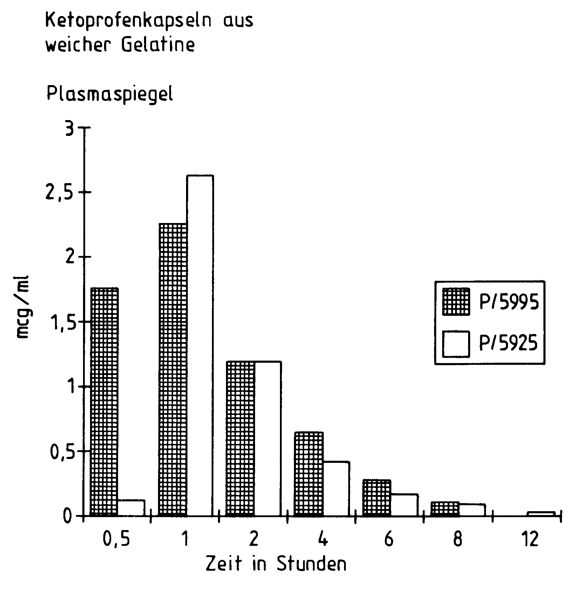

Das Säulendiagramm, das in der beigefügten Figur dargestellt ist, zeigt die Resorptionskinetik bezüglich einer pharmazeutischen Zubereitung gemäß Beispiel 1 der Erfindung (P/5995) im Vergleich zu einer Resorptionskinetik einer Zubereitung in einem Träger auf Fettbasis (P/5925). Aus dem Vergleich geht deutlich die andersartige Bioverfügbarkeit (mehr als das dreifache) des Ketoprofens hervor, das gemäß vorliegender Erfindung zubereitet wurde, vorallem innerhalb der ersten 30 Minuten.

In nachfolgender Tabelle sind die Stabilitätsdaten hinsichtlich einer Zubereitung des in Beispiel 1 beschriebenen Typs aufgeführt.

| **Test** | Bedingungen | Nach 3 Monaten in bernsteinfarbenem Glas | Nach 3 Monaten in PVC/PVDC Blister |
|---|---|---|---|
| Aspekt | 25%C/60%UR | unverändert | unverändert |
| | 30°C/70%UR | unverändert | unverändert |
| Zersetzungszeit | 25°C/60%UR | 3′ - 3′30˝ | 3′ - 3′ 40˝ |
| T₀ = 3′ - 3′20˝ | 30°C/70%UR | 2′50˝ - 3′10˝ | 3′10˝ - 3′45˝ |
| Auflösung - 30′ (sec. FU IX) | 25°C/60%UR | 96.2 % | 94.9% |
| T₀ = 97.0 % | 30°C/70%UR | 95.4% | 98.0% |
| Analoge Stoffe | 25°C/60%UR | Spuren | Spuren |
| T₀ = Spuren | 30°C/70%UR | Spuren | Spuren |
| Ketoprofen | 25°C/60%UR | 23.5 mg/cps | 23.8 mg/cps |
| T₀ = 23.7 mg/cps | 30°C/70%UR | 23.4 mg/cps | 24.0 mg/cps |

## Patentansprüche

1. Pharmazutische Zubereitung zur oralen Verabreichung mit erhöhter Bioverfügbarkeit eines Analgetikums, Antiphlogistikums, dadurch gekennzeichnet, daß sie aus einer weichen Gelatinkapsel besteht, die im wesentlichen eine therapeutisch wirkende Menge in Polyethylenglykol gelöstem Ketoprofen enthält.

2. Pharmazeutische Zubereitung gemäß Anspruch 1, dadurch gekennzeichnet, daß das Polyethylenglykol ein mittleres Molekulargewicht zwischen 200 und 600 aufweist.

3. Pharmazeutische Zubereitung gemäß einer der vorhergehenden Ansprüche, dadurch gekennzeichnet, daß das Verhältnis Ketoprofen:Polyethylenglykol zwischen 1:8 und 1:2 liegt.

4. Pharmazeutische Zubereitung gemäß einer der Ansprüche 1 oder 2, dadurch gekennzeichnet, daß die Ketoprofenlösung in Polyethylenglykol einen pH-Wert zwischen 5 und 7 aufweist.

5. Pharmazeutische Zubereitung gemäß der Ansprüche 1 bis 4, dadurch gekennzeichnet, daß das Polyethylenglykol eine Mischung von zwei oder mehreren Polyethylenglykolen ist, von denen jedes einzelne ein mittleres Molekulargewicht zwischen 200 und 600 aufweist.
